# EUROPEAN PATENT APPLICATION

(11) **EP 2 704 045 A2**
(43) Date of publication of application: **05.03.2014**
(21) Application number: 13178724.4
(22) Date of filing: 31.07.2013
(51) Int. Cl.: G06F 19/00

(54) **Apparatus and method for providing medical support**

(30) Priority: 31.08.2012 JP 2012190830
(71) Applicant: Fujifilm Corporation, Minato-ku Tokyo (JP)
(72) Inventor: Ohta, Yasunori, Kanagawa (JP)
(74) Representative: Klunker . Schmitt-Nilson . Hirsch

(57) **Abstract**

When a support request comment is transmitted from an in-hospital terminal (13) of a primary care physician, a distribution server (27) transmits a support request including initial data to each support terminal (TS1, TS2) which a support medical staff uses. A time limit is set on the initial data. After the transmission of the support request, remarks of participants in a medical support consultation and examination images are distributed only to the participants' support terminals. The time limit for the initial data transmitted to the each participant's support terminal is extended. The time limit for the initial data transmitted to the support terminal of a nonparticipant is not extended. Access to the initial data on the support terminal is disabled when the time limit is reached.

## Description

### BACKGROUND OF THE INVENTION

### 1. Field of the invention

The present invention relates to an apparatus and method for providing medical support.

### 2. Description Related to the Prior Art

A doctor often performs treatment of a patient with an injury or disease not of his/her specialty. For example, in rural areas, there are many hospitals and clinics only with generalist physicians (hereinafter simply referred to as the generalists) that do not have their specialties. It is common for those generalists to treat patients with injury or disease not of their specialties. In an emergency department, a doctor cannot foresee the type of injury or disease that patient has. It is inevitable for a duty doctor to treat an injury or disease not of his/her specialty. It is often difficult for a doctor to perform appropriate treatment for the injury or disease which is not of his/her the specialty.

A medical support system which solves the above problem is known. The medical support system allows a specialist physician (hereinafter simply referred to as the specialist) to provide medical support to a doctor who needs it. The medical support includes advice related to diagnoses and treatment, for example. In the medical support system, a terminal of a requester (primary care physician) of medical support and terminals of support medical staffs capable of participating in the medical support are connected through a network. The medical support is provided to the requester through the medical support apparatus. The medical support apparatus comprises a data producing section and a distribution section. The data producing section produces data necessary for the medical support. The distribution section distributes or transmits the data to the terminals. The medical support system distributes or transmits medical information through a communication means such as the Internet. The medical information includes findings of a primary care physician of a hospital, a treatment history, a result of an examination, an examination image, and an opinion of a specialist. With the use of the medical support system, the primary care physician can perform treatment and the like based on the advice from the specialist.

The medical information includes personal information such as age, gender, and clinical history, so that it must be handled appropriately. Generally, to prevent information leak, it is known to set a time limit on distributed data, and delete the distributed data or disable reproduction of the distributed data when the time limit is reached (see Japanese Patent Laid-Open Publication No. 2011-023024).

The time limit is set on the medical information in the Japanese Patent Laid-Open Publication No. 2011-023024, so that access to the medical information is disabled when the time limit is reached. However, when the medical information is distributed without choosing the destination(s), the distribution of the medical information to the doctors and the like not participating in the medical support consultation is continued. It is difficult to know the doctors and the like capable of participating in the medical support consultation in advance. It is also extremely inefficient to check who will be able to participate in the medical support consultation in advance.

### SUMMARY OF THE INVENTION

An obj ect of the present invention is to provide an apparatus and a method for providing medical support capable of protecting personal information of a patient and facilitating a request for the medical support.

To solve the above and other objects, the apparatus for providing medical support of the present invention comprises a first information generator, a time limit setter, a second information generator, a distributor, and a distribution controller. The first information generator produces first medical information to be transmitted at the beginning of medical support consultation for the medical support. The time limit setter sets a time limit on the first medical information. Access to the first medical information on a terminal to which the first medical information is transmitted is disabled when the time limit is reached. The second information generator produces second medical information after the first medical information. The distributor transmits the first medical information on which the time limit has been set and the second medical information. The distribution controller controls the distributor to transmit the first medical information to first terminals and transmit the second medical information to at least one second terminal out of the first terminals. The at least one second terminal transmits notification of participation in the medical support consultation after the transmission of the first medical information.

It is preferable that the first terminals are used by registered support medical staffs.

It is preferable that the first terminals are used by support medical staffs satisfying a requirement specified by a primary care physician, out of the registered support medical staffs, and the primary care physician requests the medical support.

It is preferable that the apparatus further comprises an authentication section for judging whether a user of the second terminal is the registered support medical staff based on authentication data transmitted from the second terminal. The distribution controller transmits the second medical information to the second terminal whose user has been judged as the registered support medical staff by the authentication section.

It is preferable that the apparatus further comprises a time limit extender for extending the time limit set on the first medical information in the second terminal.

It is preferable that the apparatus further comprises an information deletion section for deleting the first medical information in the first terminal not transmitting the notification of participation within a predetermined time period.

It is preferable that the first medical information is transmitted together with a support request for requesting the participation in the medical support consultation.

A method for providing medical support comprises a first production step, a setting step, first transmission step, a second production step, and a second transmission step. In the first production step, first medical information to be transmitted at the beginning of medical support consultation for the medical support is produced. In the setting step, a time limit on the first medical information is set to disable access to the first medical information on a terminal, to which the first medical information is transmitted, when the time limit is reached. In the first transmission step, the first medical information on which the time limit has been set is transmitted to first terminals. The first thermals is used by registered support medical staffs. In the second production step, second medical information after the first medical information is produced. In the second transmission step, the second medical information is transmitted to at least one second terminal out of the first terminals. The at least one second terminal transmits notification of participation in the medical support consultation after transmission of the first medical information.

According to the present invention, a time limit is set on the first medical information that is distributed or transmitted along with the support request. The first medical information is transmitted to each terminal selected or determined in advance by the operation of the primary care physician, for example. The second medical information that is transmitted after the transmission of the first medical information is transmitted to the terminal(s) which sent the notification of participation. Access to the first medical information is disabled, even through the terminal to which the first medical information is transmitted, when the time limit is reached. The second medical information that includes the personal information is transmitted only to the terminals which sent the notification of participation, so that unnecessary distribution of the second medical information is prevented. Thereby, the personal information of the patient is protected while the request for the medical support is transmitted or started easily.

### BRIEF DESCRIPTION OF THE DRAWINGS

The above and other objects and advantages of the present invention will be more apparent from the following detailed description of the preferred embodiments when read in connection with the accompanied drawings, wherein like reference numerals designate like or corresponding parts throughout the several views, and wherein:
Fig. 1 is a schematic drawing of an example of a medical support system;
Fig. 2 is a schematic drawing illustrating a registered support medical staff database;
Fig. 3 is a schematic drawing illustrating a destination database;
Fig. 4 is a block diagram illustrating a function of a medical support apparatus;
Fig. 5 is a block diagram illustrating a function of a support terminal at the time of requesting medical support;
Fig. 6 illustrates a support request screen displayed on an in-hospital terminal;
Fig. 7 illustrates a support request receiving screen of a support terminal;
Fig. 8 is an explanatory view of a display on a remark bulletin board of the support terminal;
Fig. 9 illustrates a remark input screen of the support terminal;
Fig. 10 illustrates an examination image display screen of the support terminal;
Fig. 11 is an explanatory view illustrating an electronic chart and a remark bulletin board displayed on the in-hospital terminal;
Fig. 12 is a flowchart illustrating steps for requesting medical support;
Fig. 13 is a flowchart illustrating steps for the medical support consultation;
Fig. 14 is a flowchart illustrating steps for a process performed by the time limit controller in the support terminal; and
Fig. 15 is a flowchart illustrating an example in which initial data stored in the support terminal not participating in the medical support consultation is deleted.

### DESCRIPTION OF THE PREFERRED EMBODIMENTS

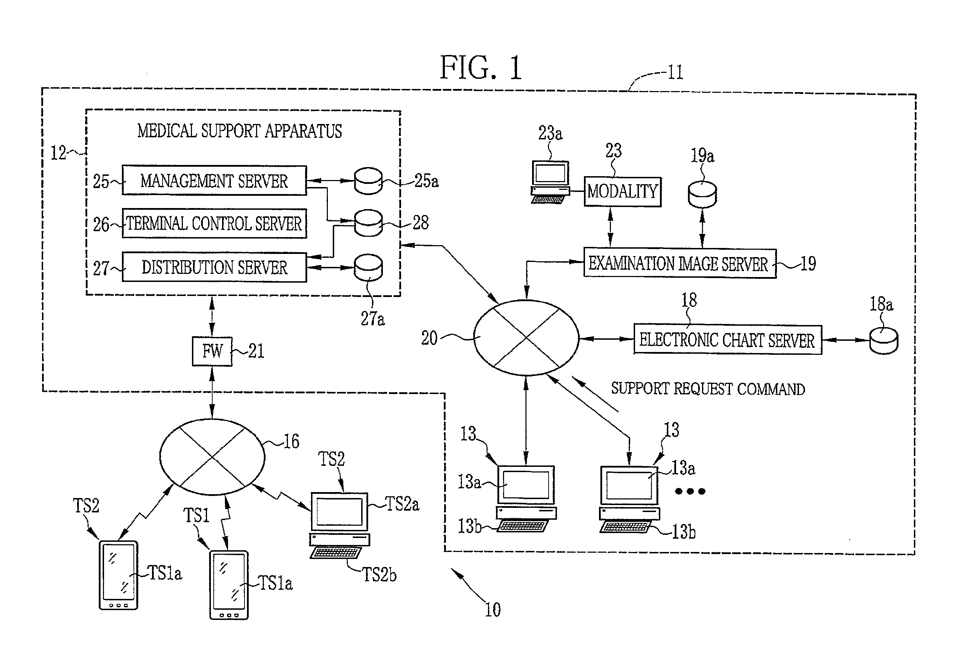

In Fig. 1, a medical support system 10 according to an embodiment of the present invention allows a registered medical staff (hereinafter referred to as a registered support medical staff) to provide medical support related to diagnosis, treatment, medical tasks, and the like to a primary care physician. For an outpatient, the primary care physician refers to a doctor who performs initial treatment or examination, for example. For an inpatient, the primary care physician refers to a doctor in charge of the treatment of the patient, for example. The medical support system 10 comprises an in-hospital subsystem 11 and support terminals (TS1 and TS2) connected to the in-hospital subsystem 11. The in-hospital subsystem 11 is built in a hospital and connected to a medical support apparatus 12 through which the medical support is provided.

In the medical support system 10, a medical support consultation is held using the medical support apparatus 12 when a primary care physician of the hospital requests the medical support through an in-hospital terminal 13 of the in-hospital subsystem 11. The medical support consultation is held per case of a patient, being an outpatient, an inpatient, or an emergency patient delivered by ambulance, for example. The medical support consultation is held to discuss and exchange medical information pertaining to the disease of the patient to provide the medical support for the patient. The primary care physician who requested the medical support and a registered support medical staff who received the support request exchange findings, remarks, and instructions on treatment of the patient while referring to medical information of the patient through their respective terminal devices.

The medical support in this embodiment includes diagnosing and treatment performed by the registered support medical staff in place of a primary care physician and advice on the diagnosis and treatment from the registered support medical staff to the primary care physician, reporting the condition of the patient, treatment based on an instruction of a doctor, arranging facilities for an examination, treatment, a medical procedure, hospitalization, or the like, and communication related to an examination, treatment, a medical procedure, hospitalization, or the like. The registered support medical staff may be any medical or health personnel, for example, a doctor, a nurse, or a lab technologist. The registered support medical staff may include a hospital clerk. Note that, in this example, the support medical staff registered with the medical support apparatus 12 in advance is referred to as the registered support medical staff. The support medical staff participating in a medical support consultation is referred to as the participant.

A tablet terminal TS1 carried by the registered support medical staff, a personal computer TS2 in home or office of the registered support medical staff, or the like is used as the support terminal TS. A smartphone installed with an application (hereinafter referred to as the support application) exclusive for the medical support system is used as the tablet terminal TS1. The tablet terminal TS1 is provided with a display TS1a that displays various screens for the medical support consultation. The display TS1a is a touch screen. Various commands for the medical support consultation or the like, and letters are inputted with a touch on the display TS1a.

The personal computer TS2 is installed with the support application. A monitor TS2a of the personal computer TS2 displays various screens for the medical support consultation. Various commands for the medical support consultation or the like, and text are inputted using a keyboard TS2b and a mouse.

Note that the support terminal TS, being the terminal device for the registered support medical staff, may be any device having a communication function and capable of displaying various screens and allowing input as necessary. Various mobile terminal devices such as a smartphone, a mobile phone, and a notebook PC can be used as the support terminals TS. The support terminals TS may include the in-hospital terminal 13. Namely, the registered support medical staff who belongs to the same hospital as that of the primary care physician may provide the medical support using the in-hospital terminal 13.

The support terminal TS is connected to the in-hospital subsystem 11 through a network 16. Any type of network can be used as long as it connects the support terminal TS and the in-hospital subsystem 11 in a communicable manner regardless of whether the support terminal TS is located within or outside of the hospital. The network 16 is, for example, the Internet, an exclusive line, a mobile phone line, a public wireless LAN, or a combination thereof. Note that it is preferable to connect the support terminal TS and the in-hospital subsystem 11 through VPN (Virtual Private Network) to prevent information leak.

The in-hospital subsystem 11 is composed of the medical support apparatus 12, the in-hospital terminal 13, an electronic chart server 18 and an examination image server 19 for providing medical information, an in-hospital LAN 20, a firewall 21, and the like. The medical support apparatus 12, the in-hospital terminal 13, the electronic chart server 18, the examination image server 19 are connected through the in-hospital LAN 20. The firewall 21 is provided between the network 16 and the medical support apparatus 12. As for the servers for providing the medical information, the servers 18 and 19 are used by way of example. A server is used depending on the data to be provided. For example, a server for providing results of a blood test or a pathologic examination may be used.

The in-hospital LAN 20 is a network built in the hospital using the Internet technology. The in-hospital LAN connects the medical support apparatus 12, the in-hospital terminal 13, and the servers 18 and 19 in a communicable manner. The firewall 21 permits only the communication for the medical support consultation between the support terminal TS and the medical support apparatus 12 through the network 16. The firewall 21 blocks unauthorized access to the medical support apparatus 12 and other devices connected to the in-hospital LAN to prevent the information leak and alteration of data.

The in-hospital terminals 13 are disposed in examination rooms and treatment rooms of various departments, for example, surgery and internal medicine, and an emergency center in a hospital. The in-hospital terminal 13 is a personal computer on which an application is installed. The application includes a program for electronic charts, a program for requesting examination, and a program for the medical support. A monitor 13a of the in-hospital terminal 13 displays an electronic chart, a medical image, a support request screen for requesting the medical support, and various screens for the medical support consultation. A keyboard 13b and a mouse (not shown) of the in-hospital terminal 13 are used to refer to or input an electronic chart, request an examination such as X-ray imaging, refer to medical images, request the medical support, and input remarks during the medical support consultation, for example. By accessing the medical support apparatus 12 through the in-hospital terminal 13, data of a registered support medical staff is deleted or registered.

The in-hospital terminal 13 sends a support request command to the medical support apparatus 12 to request the medical support. The support request command is generated based on the setting and input to the support request screen. The support request command includes data for specifying destination(s) of the support request. Upon receiving the support request command from the in-hospital terminal 13, the medical support apparatus 12 starts the medical support consultation and distributes or delivers the support request to the registered support medical staffs to request the registered support medical staffs to participate in the medical support consultation. The support request includes the medical information. The registered support medical staffs are included in a medical support group.

The electronic chart server 18 stores initial data and treatment data of each patient in an electronic chart database 18a and provides the data as necessary. The electronic chart server 18 stores the initial data as a initial data record in the electronic chart database 18a. The initial data includes the name, the date of birth, gender, the weight, the height, and the like of a patient. The initial data record also includes a patient ID assigned to each patient. The initial data record is identified with the patient ID. A new initial data record is created for a new patient (patient with no patient ID) and stored in the electronic chart database 18a by inputting the initial data through a terminal (not shown) for new registration.

The electronic chart server 18 stores the treatment data, inputted to the electronic chart, as a treatment data record in the electronic chart database 18a. A registration ID and the patient ID are stored in the treatment data record. The registration ID is issued by a registration terminal (not shown) at the start or the registration of treatment. The treatment data includes history of treatment, for example, findings and prescription, inputted to the electronic chart, examination data, vital signs, and a doctor ID of the primary care physician. When the treatment data includes an examination image, its ID (image ID) is also stored in the treatment data record.

A modality 23, such as a CT device or an MRI device is connected to the examination image server 19. The examination image server 19 stores an examination image, such as a CT image obtained from the modality 23, in an examination image database 19a and provides the image as necessary. The examination image server 19 receives a request (examination request) of examination from the in-hospital terminal 13. The examination image server 19 displays an instruction corresponding to the examination request on an operation terminal 23a of the modality 23. An examination image of the patient taken with the modality 23 by a technologist is stored as the examination image record in the examination image database 19a. The examination image record includes an image ID for identification of the examination image, an imaging time, a technologist ID assigned to the technologist who took the examination image, the registration ID, and the patient ID, in addition to the examination image.

When the medical support consultation is started, the medical support apparatus 12, for example, notifies the electronic chart server 18 and the examination image server 19 of the registration ID and the patient ID of the case of the patient, being the subject of the medical support consultation. Thereafter, the electronic chart server 18 sends the updated content of the electronic chart corresponding to the registration ID to the medical support apparatus 12 when the content of the electronic chart is updated. When a new examination image corresponding to the patient ID is taken, the examination image server 19 sends the examination image to the medical support apparatus 12. Thereby, the updated content of the electronic chart and the new examination image are distributed or transmitted automatically. Note that the primary care physician may manually transmit the updated content of the electronic chart and the new examination image.

The medical support apparatus 12 is composed of a management server 25, a terminal control server 26, a distribution server 27, and the like. The management server 25 registers a support medical staff or cancels the registration of a registered support medical staff, authenticates a registered support medical staff before or when he/she participates in the medical support, and manages participation of the registered support medical staff in the medical support consultation. A registered support medical staff database 25a is connected to the management server 25. The registered support medical staff database 25a stores data related to the registered support medical staffs.

By accessing the management server 25 through the in-hospital terminal 13 or a management terminal (not shown), a new support medical staff is registered, data of a registered support medical staff is changed, or the registration of a registered support medical staff is canceled. When a new support medical staff is registered, the management server 25 creates a new registered support medical staff record and stores it in the registered support medical staff database 25a. When the registration of a registered support medical staff is cancelled, the management server 25 deletes the corresponding registered support medical staff record from the registered support medical staff database 25a.

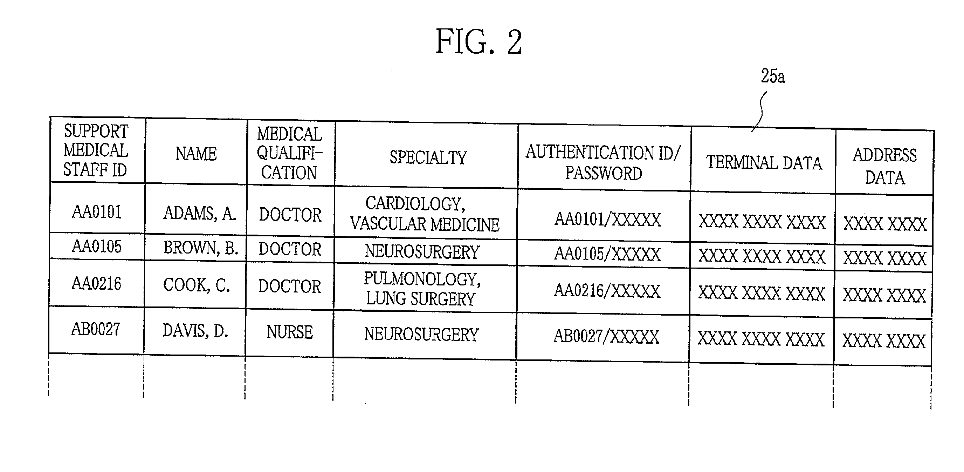

As shown in Fig. 2, a registered support medical staff ID for identifying the registered support medical staff, attribute information of the registered support medical staff, an ID (authentication ID) and a password for authentication, data (terminal data) of the terminal in use, address data of the terminal, and the like are stored in each registered support medical staff record in the registered support medical staff database 25a. The attribute information includes the name of the registered support medical staff, data of his/her medical qualification or job title such as "doctor", "nurse", "technologist", "pharmacist", or the like, and data (specialty data) of his/her specialty. The specialty data specifies the registered support medical staff's specialty or the department to which the registered support medical staff belongs. Examples of the specialty data include "cardiology and vascular medicine (heart and blood vessels)" "neurosurgery (brain surgery)", "pulmonology (respiratory medicine) and lung surgery", "radiographic interpretation", and the like.

The terminal data is used to identify the support terminal TS which the registered support medical staff uses. For example, a MAC (Media Access Control) address assigned to a network interface of the support terminal TS or a specific number called "IMSI (International Mobile Subscriber Identity) " stored in a SIM (Subscriber Identity Module) card for mobile phone in the tablet terminal TS1 is used as the terminal data. The address data indicates a destination or address of the terminal in use, for example, an IP address, a mail address, or a phone number of a mobile phone to which information or data is sent.

Note that the registered support medical staff records with different terminal data and address data may be stored for a single registered support medical staff so as to make two or more support terminals TS available for the registered support medical staff. The support terminal TS may be provided with different types of terminal data such as the MAC address and the IMSI. The terminal data compliant with the communication method is used for the authentication of the support terminal TS to participate in the medical support consultation.

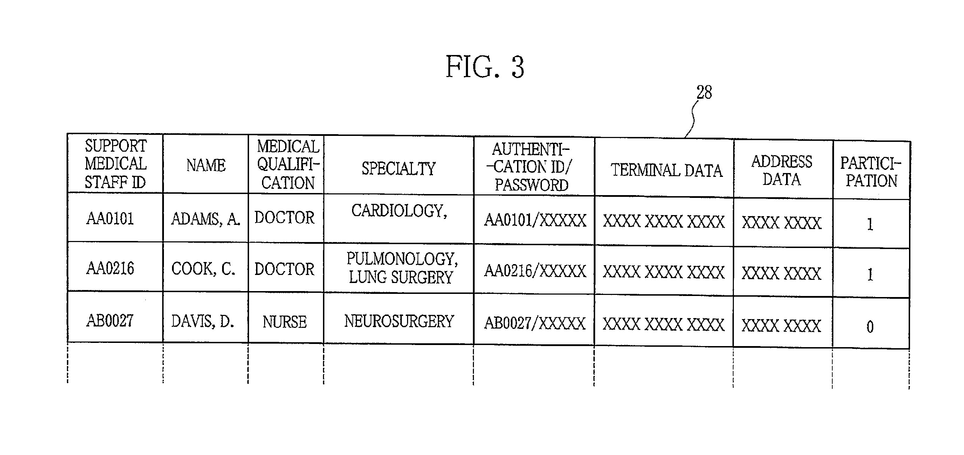

A destination database 28, in which the destination records are stored, is connected to the management server 25. As shown in Fig. 3, the destination record in the destination database 28 is composed of the registered support medical staff record and a column (participation column) for "participation". The destination of each data, sent from the distribution server 27, is controlled by recording or storing the destination record in the destination database 28 and making a change in the participation column.

The terminal control server 26 controls the support terminal TS through the support application. For example, the terminal control server 26 deletes delivered data stored in the support terminal TS when a single medical support consultation is ended. The terminal control server 26 extends a time limit for referring to the initial data stored in the support terminal TS. The initial data (support request data) is included in the support request distributed or delivered by the distribution server 27.

The distribution server 27 distributes or transmits various types of information pertaining to the medical support consultation to the support terminals TS. The information includes the request (support request) for the medical support. The distribution server 27 produces the support request based on the support request command from the in-hospital terminal 13. The support request includes the initial data. The distribution server 27 sets a time limit on the initial data. Access to the initial data on the support terminal TS is disabled when the time limit is reached. After the support request is transmitted, the distribution server 27 transmits comments and remarks from the primary care physician and the participants, the updated content of the electronic chart, a new examination image, or the like to each of the in-hospital terminal 13 of the primary care physician and the support terminals TS.

A delivered information database 27a is connected to the distribution server 27. The delivered information database 27a stores a delivered data record. The delivered data record includes the information or data distributed or transmitted to the support terminal TS and the in-hospital terminal 13 during the medical support consultation and the initial data. To be more specific, the delivered data record includes the registration ID, a delivery ID for identifying the data delivered, a delivery time, a time of receiving the data (a remark or an examination image) that is the source of the data delivered, an ID (the primary care physician's ID or the support medical staff's registered support medical staff ID) of a speaker who inputted a remark, content of the data delivered, and the like. When the delivered data is a remark, the content of the remark is stored in the delivered data record. When the delivered data is an examination image, its image ID and a device ID of the modality 23 used for imaging the examination image are stored in the delivered data record.

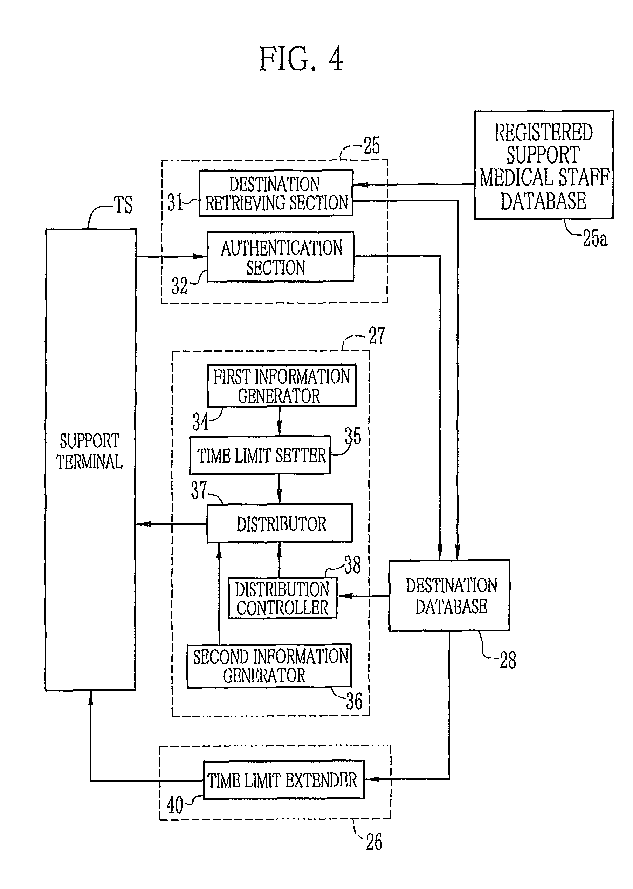

As shown in Fig. 4, the management server 25 functions as a destination retrieving section 31 and an authentication section 32. In response to the support request command from the in-hospital terminal 13, the destination retrieving section 31 retrieves the registered support medical staff record of the registered support medical staff corresponding to the destination selected or specified by the primary care physician through the in-hospital terminal 13. The destination retrieving section 31 adds the participation column to the retrieved registered support medical staff record and stores the retrieved registered support medical staff record with participation column as the destination record in the destination database 28. Whether the registered support medical staff record matches the destination selected or specified is judged based on the medical qualification data and the specialty data in the registered support medical staff record. The registered support medical staff record which matches a requirement specified as the destination is retrieved from the registered support medical staff database 25a.

Based on authentication data transmitted from the support terminal TS, the authentication section 32 judges whether the user of the support terminal TS is a valid registered support medical staff allowed to participate in the medical support consultation. To be more specific, the authentication section 32 authenticates the user as the valid registered support medical staff based on the authentication data, being an authentication ID, a password, and the terminal data, transmitted together with the participation notification from the support terminal TS when the user participates in the medical support consultation after the distribution or transmission of the support request. In this authentication, the destination record which matches the transmitted terminal data is retrieved or identified in the destination database 28. The user is authenticated as the valid registered support medical staff when the authentication ID and the password stored in the identified destination record match those transmitted from the support terminal TS. The user authenticated as the valid registered support medical staff is allowed to participate in the medical support consultation. The authentication section 32 writes a participation flag "1" in the participation column, indicating that this registered support medical staff participates in the medical support consultation. Note that application for the participation in the medical support consultation may be allowed for a certain period of time. Namely, the authentication section 32 authenticates the user(s) only within a predetermined period of time after the support request is delivered.

The distribution server 27 functions as a first information generator 34, a time limit setter 35, a second information generator 36, a distributor 37, and a distribution controller 38. The fist information generator 34 produces initial data based on the information or data included in the support request command. The initial data is the first medical information to be transmitted initially or at the beginning of the medical support consultation. The initial data includes a patient ID of the patient, being the subject of the medical support consultation, the registration ID and the registration time of the case of the patient, a support request comment inputted by the primary care physician, one or more examination images taken with the modality 23 between the acceptance of the patient and the transmission of the support request command, and the content of the electronic chart pertaining to the case. The distribution server 27 retrieves the content of the electronic chart, necessary for producing the initial data, from the electronic chart server 18 using the registration ID as a key, for example. The distribution server 27 retrieves the examination image(s), necessary for producing the initial data, from the examination image server 19 using the image ID as the key, for example.

The time limit setter 35 sets a time limit or expiration time on the initial data. The time limit setter 35 prohibits displaying the initial data on the display TS1a or the monitor TS2a when the time limit for the support application of the support terminal TS is reached. For example, the initial data is encoded and transmitted to the support terminal TS. Within the time limit, the support application decodes the initial data and displays the decoded initial data on the display TS1a or the monitor TS2a. When the time limit is reached, the initial data is not decoded. For example, the time limit is 10 minutes after the distribution of the initial data or the support request. Note that the encoding prevents direct access to the content of the initial data without using the support application.

The second information generator 36 produces standard data, being second medical information distributed or transmitted after the transmission of the first medical information, namely, after the support request is transmitted. The second information generator 36 receives remarks from the participants through their respective support terminals TS, a remark from the primary care physician that transmitted the support request through the in-hospital terminal 13, the updated content of the electronic chart from the electronic chart server 18, and a new examination image from the examination image server 19. The second information generator 36 adds additional data to the remark, the updated content, the examination image, and the like to produce the standard data.

The additional data for the remark includes the time of inputting the remark and the name of the speaker who inputted the remark, for example. The name of the speaker is identified from the information received with the remark. Namely, the name of the speaker is the name of the registered support medical staff corresponding to the support terminal TS which transmitted the remark. The name of the registered support medical staff and the data of the support terminal TS are found in the destination database 28. The additional data for the examination image includes the examination ID, the imaging time, and the modality used. Note that the examination image is reduced to an appropriate size.

Under the control of the distribution controller 38, the distributor 37 distributes or transmits the initial data to which the time limit has been set and standard data produced by the second information generator 36. Based on the destination record in the destination database 28, the distribution controller 38 controls the distribution or delivery when and after the support request is transmitted. The distribution controller 38 allows the distributor 37 to transmit the support request to an address indicated by the address data in each destination record in the destination database 28, for example. Thereby the medical support request is delivered to all the support terminal(s) TS corresponding to the support medical staff(s) (destination) appointed or selected by the primary care physician through the in-hospital terminal 13. After the support request is transmitted, the standard data is transmitted to the address indicated by the address data in each destination record in which the participation flag "1" is written, out of the destination records stored in the destination database 28. Thereby, the standard data is transmitted only to the registered support medical staff or the participant who has expressed participating in the medical support consultation and has been authenticated.

The terminal control server 26 functions as a time limit extender 40. The time limit extender 40 extends the time limit set on the initial data stored in the support terminal TS of the participant in the medical support consultation. Within the time limit, access to the initial data is permitted. After the medical support consultation is started, the time limit extender 40 accesses the destination database 28 at appropriate intervals to monitor the participation column of each destination record. Upon detecting a new destination record with the participation flag "1", the time limit extender 40 accesses the support terminal TS corresponding to the new destination record and extends the time limit for the initial data stored in the support terminal TS. The time limit may be extended as necessary. For example, the time limit is extended for three more hours. Note that the time limit may be extended again if the medical support consultation is continuing beyond the extended time limit.

Note that Fig. 4 illustrates how the support request and the standard data are distributed or delivered from the medical support apparatus 12 to the support terminal TS. The standard data is transmitted unconditionally to the primary care physician's in-hospital terminal 13 through which the support request command is transmitted.

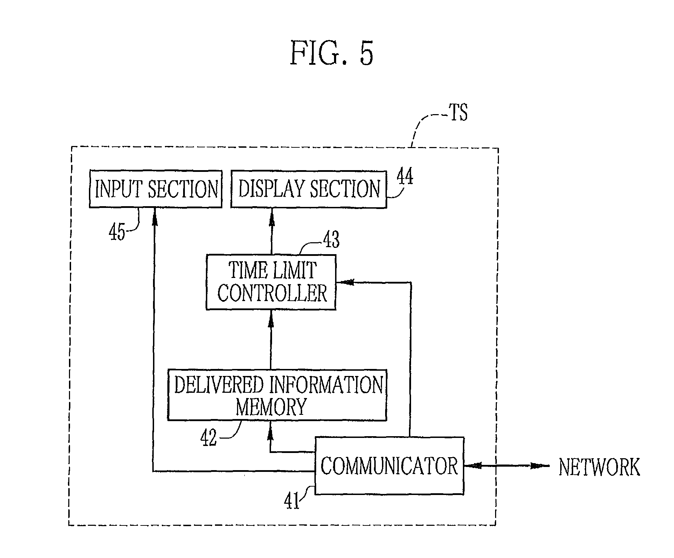

The support terminal TS is installed with the support application as described above. As shown in Fig. 5, execution of the support application allows the support terminal TS to function as a communicator 41, a delivered information memory 42, a time limit controller 43, a display section 44, and an input section 45. The communicator 41 is composed of a communication circuit such as a network interface and the like. The communicator 41 communicates with the medical support apparatus 12 through the network 16 and the firewall 21.

The initial data and the standard data, distributed or transmitted from the medical support apparatus 12, is written into and stored in the delivered information memory 42. The time limit extender 40 accesses the delivered information memory 42. The limit extender 40 extends the time limit for the initial data stored in the delivered information memory 42.

The display section 44 of the tablet terminal TS1 is composed of the display TS1a and the like. The display section 44 of the personal computer TS2 is composed of the monitor TS2a and the like. The display section 44 accesses the delivered information memory 42 through the time limit controller 43 to retrieve the initial data and the standard data. The display section 44 displays various screens of the medical support consultation based on the retrieved data. The time limit controller 43 compares the time limit set on the initial data with the current time and date before the display section 44 displays a screen based on the initial data. When the current time and date is within the time limit, the time limit controller 43 decodes the encoded initial data, transmits decoded initial data to the display section 44, and allows the display section 44 to display the screen based on the initial data. When the current time and date is the same as those of the time limit or exceed the time limit, the initial data is not decoded. Hence, the time limit controller 43 does not allow the display section 44 to display the screen based on the initial data. Note that the access to the initial data by the display section 44 may be disabled on and after the time limit so that the initial data will not be displayed.

The input section 45 of the tablet terminal TS1 is composed of a touch screen on a display TS1a and the like. The input section 45 of the personal computer TS2 is composed of the keyboard TS2b, the mouse, and the like. The support terminal TS receives the input from the input section 45. A registered support medical staff operates the input section 45 to participate in the medical support consultation, to input a remark during the medical support consultation, and to refer to the examination image, for example.

At least a part of the function of the above-described support application of the support terminal TS is always kept turned on so as to receive the support request from the medical support apparatus 12 when the support terminal TS is on or in a "sleep" mode. In response to receiving the support request through the communicator 41, the display section 44 displays the support request receiving screen. Thereby, the support request is immediately displayed on the support terminal TS of the registered support medical staff. The registered support medical staff immediately corresponds to the support request of an urgent case. Note that it is preferable to use sound, vibration, or the like to notify the registered support medical staff of the reception of the support request.

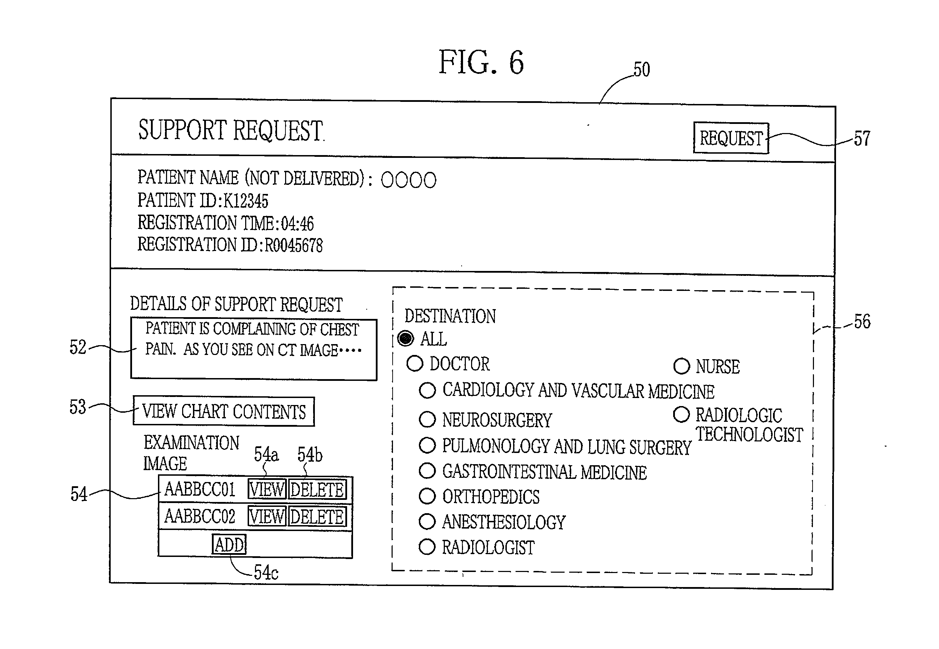

Next, screens displayed on the in-hospital terminal 13 and the support terminal TS during the medical support consultation are described. In Fig. 6, the monitor 13a of the in-hospital terminal 13 displays a support request screen 50. The support request screen 50 is displayed, for example, by a predetermined operation on an electronic chart screen of the in-hospital terminal 13. The support request command generated based on the setting and input to the support request screen 50 is sent from the in-hospital terminal 13 to the medical support apparatus 12.

The support request screen 50 displays at least a part of the contents of the electronic chart of a patient, being the subject of the medical support. The patient name, the patient ID, the registration time, and the registration ID displayed on the support request screen 50 are the same as those displayed on the electronic chart screen that displays the electronic chart of the case of the patient. Note that the patient ID, the registration time, and the registration ID are delivered together with the support request from the distribution server 27 to each support terminal TS. The patient name, being information to identify an individual, is not delivered.

The primary care physician inputs a support request comment to a comment box 52. The support request comment is, for example, a comment describing a reason for requesting the medical support. When a chart content button 53 is clicked with the mouse, the in-hospital terminal 13 displays the contents of the electronic chart delivered with the support request, for example, a symptom, the primary care physician's findings, a treatment history, and the like, out of all the contents of the electronic chart.

The support request screen 50 displays an examination image list 54. The examination image list 54 is a list of image IDs of the examination images to be delivered by the distribution server 27. The distribution server 27 delivers the examination image list together with the support request. When the support request screen 50 is initiated, the in-hospital terminal 13 automatically accesses the examination image server 19, using the registration ID as a key. The in-hospital terminal 13 retrieves the image IDs of the examination images, pertaining to the case, taken between the acceptance of the patient and the startup of the support request screen 50. The retrieved image IDs are displayed on the examination image list 54.

A view button 54a and a delete button 54b are displayed for each image ID in the examination image list 54. With a click on the view button 54a, the in-hospital terminal 13 retrieves a corresponding examination image from the examination image server 19 and displays it on the display. With a click on the delete button 54b, the corresponding image ID is deleted from the examination image list. Namely, the examination image with the deleted image ID is not delivered.

An add button 54c is displayed on the examination image list 54. The add button 54c is used to add an examination image to be delivered. With a click on the add button 54c, the in-hospital terminal 13 displays a screen (selection screen) for selecting examination image (s) pertaining to the case of the patient, being the subject of the medical support. The examination image selected on the selection screen is added as the examination image to be delivered. The image ID of the selected examination image is added to the examination image list 54. Thereby, the examination image (s) and the like pertaining to the case, out of all the examination images of the patient, are delivered.

The support request screen 50 displays a destination selection section 56 for specifying or selecting the destination to which the support request is distributed or delivered. In this example, if the support request is to be delivered to all the registered support medical staffs, "all" is clicked. Instead, the registered support medical staffs are selected on a group basis according to a medical qualification or job title, a specialty, and/or a department, for example. By selecting a destination (for example, the medical qualification, the specialty, or the department) on the destination selection section 56, one or more registered support medical staffs corresponding to the selected destination are selected. The medical support request is distributed to the selected registered support medical staff(s). As for the medical qualification, at least one of "doctor", "nurse", and/or "radiologic technologist" may be selected. As for the department, at least one of "cardiology and vascular medicine (heart and blood vessels)", "neurosurgery (brain surgery)", and/or "pulmonology (respiratory medicine) and lung surgery" may be selected. Two or more destinations (items) can be selected at a time.

In response to a click on a command button 57, the in-hospital terminal 13 sends a support request command to the medical support apparatus 12. The support request command includes the command data inputted and set through the support request screen 50. Namely, the selected destination(s) is sent to the management server 25. The registration ID, the support request comment, the image ID(s) selected from the examination image list 54 are transmitted to the to the distribution server 27. Based on the registration ID and the image ID(s), pieces of data to be delivered as the initial data are retrieved.

The display section 44 displays the support request receiving screen the display TS1a (or the monitor TS2a) when the support terminal TS receives the support request from the distribution server 27. Note that during the medical support consultation, the screen displayed on the monitor TS2a of the personal computer TS2 is the same as that displayed on the display TS1a of the tablet terminal TS1. Hereinafter, the screen displayed on the display TS1a of the tablet terminal TS1 is described by way of example.

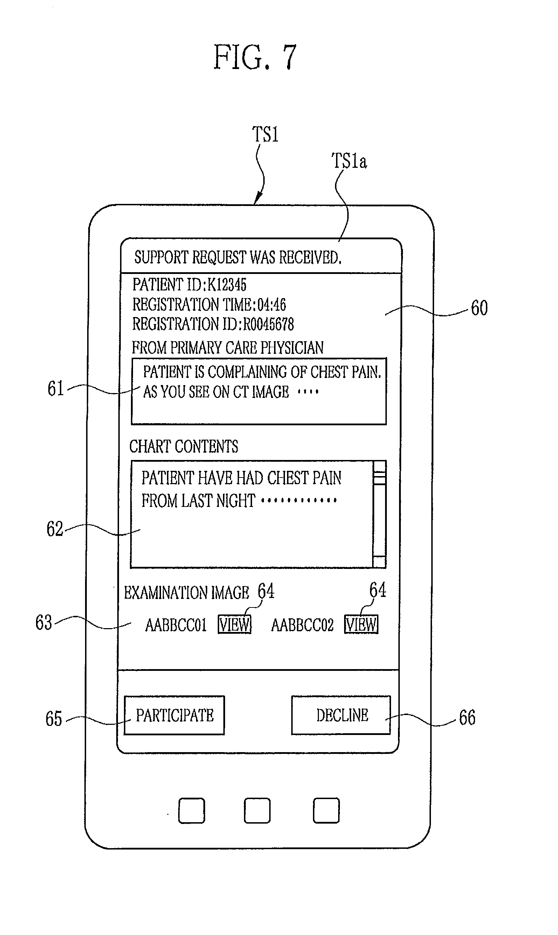

As shown in Fig. 7, the display TS1a displays the support request receiving screen 60 by way of example. The support request receiving screen 60 displays the initial data delivered from the distribution server 27. The initial data includes the patient ID, the registration time, the registration ID, a support request comment 61 inputted in the comment box 52, chart content 62 pertaining to this case in the electronic chart, and an examination image list 63 corresponding to the examination image list 54.

The display section 44 displays image view button(s) 64, a participation button 65, and a decline button 66 on the support request receiving screen 60. The image view buttons 64 correspond to the respective image IDs in the examination image list 63. With a touch on the image view button 64, an examination image display screen opens and the examination image corresponding to the image view button 64 is displayed on the display TS1a. With a touch on the participation button 65, participation notification, notifying that the registered support medical staff using this support terminal TS participates in the medical support consultation, is sent to the medical support apparatus 12. With a touch on the decline button 66, decline notification, notifying that the registered support medical staff using this support terminal TS does not participate in the medical support consultation, is sent to the medical support apparatus 12.

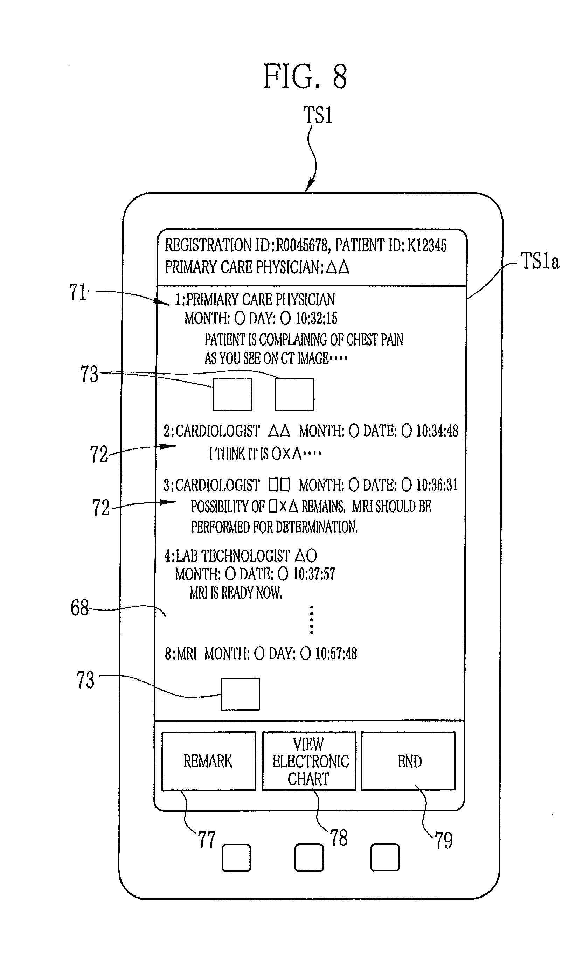

As shown in Fig. 8, when the medical support consultation is started, a remark bulletin board 68 is displayed on the display TS1a. On the remark bulletin board 68, a support request comment 71, remarks 72 of the primary care physician and the participants, a thumbnail image 73 of the examination image are arranged in time order in an electronic bulletin board method (system). The contents displayed on the remark bulletin board 68 are updated every time a remark or an examination image is distributed or transmitted.

The thumbnail images 73 of the examination images include the thumbnail image 73 of the examination image included in the initial data and the thumbnail image 73 of the examination image automatically distributed or transmitted. In this example, the thumbnail image 73 of the examination image included in the initial data is displayed along with the support request comment 71. The thumbnail image 73 is produced by reducing the size of the delivered examination image. Here, the display section 44 produces the thumbnail image by way of example. With a touch on the thumbnail image 73, the screen is switched to the examination image display screen, on which the examination image corresponding to the touched thumbnail image 73 is displayed. Note that, for example, the distribution server 27 may produce the thumbnail image 73 and transmit it together with the examination image.

Above the remark bulletin board 68, the patient ID, the registration ID, and the name of the primary care physician who sent the support request are displayed. Below the remark bulletin board 68, a remark button 77, a chart view button 78, and an end button 79 are displayed. In response to a touch on the remark button 77, the display section 44 switches the screen to a remark input screen. In response to a touch on the chart view button 78, the display section 44 switches the screen to display the distributed content of the electronic chart. The end button 79 is operated to withdraw from the medical support consultation. In response to a touch on the end button 79, withdrawal notification is transmitted to the medical support apparatus 12. In response to the withdrawal notification, the management server 25 deletes the participation flag in the destination record corresponding to the support terminal TS which transmitted the withdrawal notification. Thereby, the distribution or transmission of information to the support terminal TS is stopped.

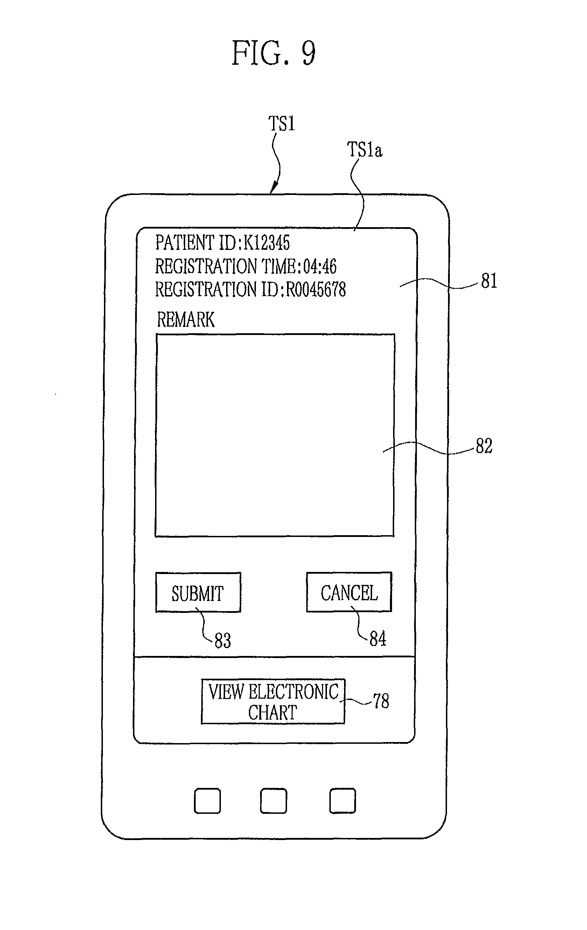

As shown in Fig. 9, a remark input screen 81 displays a remark box 82 in addition to the patient ID, the registration time, and the registration ID. A remark is inputted to the remark box 82 through the input section 45, being the soft keyboard or the like on the touch screen. In response to a touch on a submit button 83, the remark inputted to the remark box 82 is transmitted to the medical support apparatus 12 through the communicator 41. The distribution server 27 receives the remark, and transmits it to each of the participants' support terminals TS and the primary care physician' s in-hospital terminal 13. A cancel button 84 is used to close the remark input screen 81 and return to the immediately preceding screen. The remark input screen 81 displays the chart view button 78. With a touch on the chart view button 78, the participant can view the contents of the electronic chart while inputting a remark in the remark box 82. Note that the remark input screen 81 may display the image view button used for viewing the examination image.

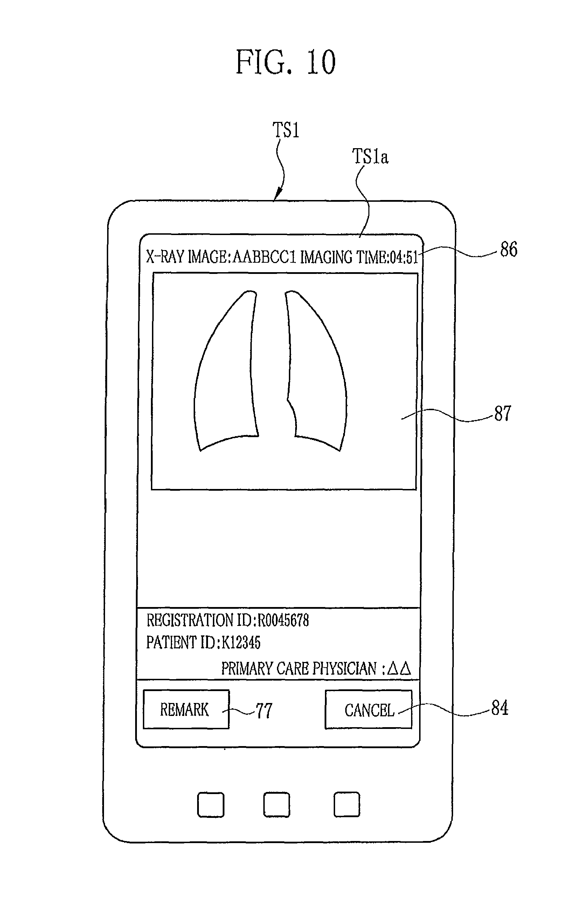

As shown in Fig. 10, an examination image display screen 86 displays the type of the examination image (for example, X-ray image, CT image, or the like), the image ID, the imaging time, and an examination image 87. The participant views the examination image 87 in detail on the examination image display screen 86. Note that a tool bar is displayed when a CT image or an MRI image is displayed. The tool bar is used to control a cross-section or an orientation of the examination image displayed. With the use of the tool bar, a desired cross-section of the examination image 87 can be viewed in a desired orientation.

The examination image display screen 86 displays the remark button 77 and the cancel button 84. The functions of the remark button 77 and the cancel button 84 in Fig. 10 are the same as those of the above-described remark button 77 and the cancel button 84 in Figs. 8 and 9. Note that the remark button 77 is not displayed when the examination image display screen is opened using the image view button 64 on the support request receiving screen 60.

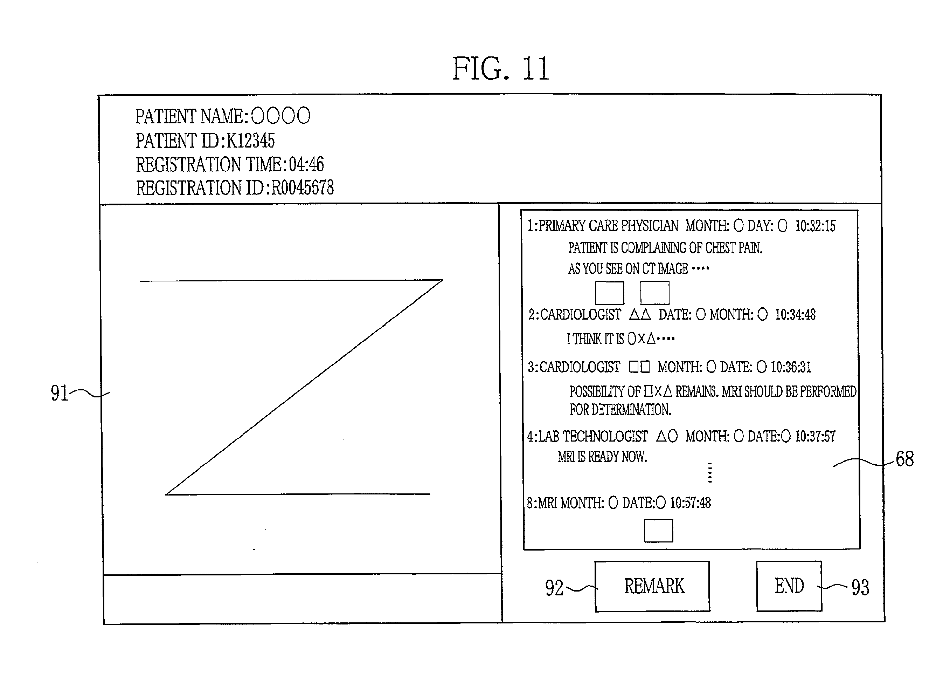

As shown in Fig. 11, after the medical support consultation is started, the monitor 13a of the in-hospital terminal 13 displays an electronic chart 91 and the remark bulletin board 68. The same remark bulletin board 68 as that displayed on the support terminal TS is displayed on the monitor 13a of the in-hospital terminal 13. Thereby, the primary care physician can share and understand the remarks of the participants easily. The monitor 13a of the in-hospital terminal 13 also displays a remark button 92 and an end button 93. With the operation of the remark button 92, the primary care physician inputs and sends a remark during the medical support consultation. The remark is transmitted from the in-hospital terminal 13 to the medical support apparatus 12 through the in-hospital LAN 20. The end button 93 is used to transmit an end notification, notifying the end of the medical support consultation, from the in-hospital terminal 13 to the medical support apparatus 12. In response to the end notification, the medical support apparatus 12 executes a predetermined end sequence to end the medical support consultation.

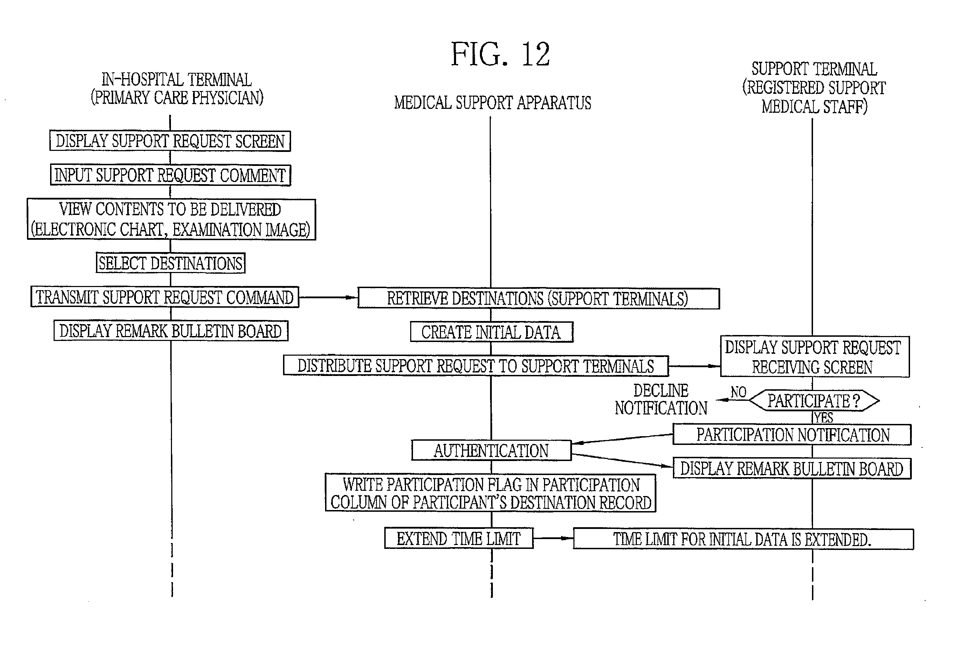

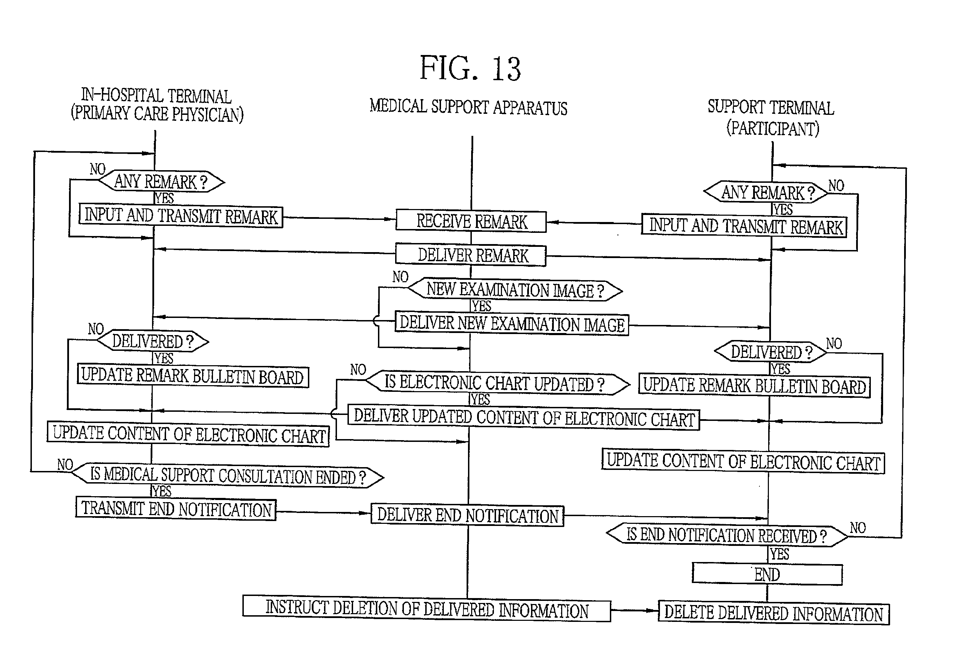

Referring to Figs. 12 and 13, an operation of the above-configured medical support system 10 is described. For example, the medical support using the medical support system 10 is requested when a primary care physician needs advice on diagnosis or treatment of a disease or an injury of a patient from a specialist or a radiologist. Note that the patient may be an outpatient, an emergency outpatient outside of regular office hours, an emergency patient delivered by ambulance, or an inpatient, for example.

For example, when the primary care physician needs the medical support on treatment (hereinafter referred to as the case) of the patient, the primary care physician operates the in-hospital terminal 13. The primary care physician performs a predetermined operation on the electronic chart screen to display the support request screen 50 shown in Fig. 6. Thereby, the support request screen 50 corresponding to the case of the patient is displayed. There is no need for the primary care physician to input or select the patient ID and the registration ID assigned at the time of the registration of the case.

After the support request screen 50 is displayed, the primary care physician inputs the support request comment to the comment box 52. The support request comment may include any type of content. For example, a reason for the support request or what the primary care physician needs advice on is inputted. The primary care physician clicks on the chart content button 53 to view and check the content of the electronic chart to be distributed or transmitted. The in-hospital terminal 13 retrieves the content to be transmitted, out of the contents of the electronic chart of the case, from the electronic chart server 18, using the registration ID as a key. The in-hospital terminal 13 displays the content retrieved. If there is content to be added to the electronic chart, the primary care physician adds the content to the electronic chart, or inputs "the content needs to be added" as the support request comment to the comment box 52.

The examination image list 54 displays the image IDs of the taken examination images, corresponding to the case, retrieved from the electronic chart. To view the examination image, the primary care physician clicks on the view button 54a. In response to this, the in-hospital terminal 13 retrieves the examination image, having the image ID corresponding to the view button 54a clicked, from examination image server 19 and displays the examination image retrieved. If the displayed examination image is unnecessary, the examination image is closed, and the delete button 54b corresponding to the displayed examination image is clicked. Thereby, the image ID of the examination image is deleted. Note that, if the examination image is to be transmitted, no additional operation is necessary after the examination image is closed.

For example, to distribute or transmit the examination image taken in the past treatment with the support request, the add button 54c is clicked. In response to this, the in-hospital terminal 13 displays a selection screen. The selection screen displays the examination images of the patient, for example, in thumbnail images, stored in the examination image database 19a. The primary care physician refers to the thumbnail images of the examination images and selects the necessary examination image. When the examination image is selected, the image ID of the selected examination image is displayed on the examination image list 54. To add two or more examination images, the above-described operation is repeated.

The primary care physician clicks on the command button 57, for example, after he/she selects the destination(s), in accordance with the content of the support, through the destination selection section 56. In response to the click, the support request command is sent from the in-hospital terminal 13 to the medical support apparatus 12. The support request command is based on the input and settings provided through the support request screen 50. The support request command has the command data including the patient ID, the registration time, the registration ID, the support request comment, and the image IDs displayed on the examination image list 54. The name of the primary care physician who sent the support request command is also transmitted together with the support request command. As shown in Fig. 11, after the support request command is transmitted, the remark bulletin board 68 is displayed on the in-hospital terminal 13.

When the medical support apparatus 12 receives the support request command, the selected destination, out of the command data, are sent to the management server 25. The destination retrieving section 31 of the management server 25 accesses the registered support medical staff database 25a and retrieves the registered support medical staff records of the registered support medical staffs included in the group specified as the destination. For example, when the "doctor" is specified as the destination, each registered support medical staff record with the medical qualification "doctor" is retrieved. When the "pulmonology and lung surgery" is specified as the destination, each of the support medical staff records of the doctors and nurses with the specialty data of "pulmonology and lung surgery" is retrieved. When the "radiologist" is specified as the destination, each registered support medical staff record of a doctor with the specialty data of "radiologist" is retrieved. Note that when "all" is specified as the destination, all the registered support medical staff records stored in the registered support medical staff database 25a are retrieved.

The participation column is added to the retrieved registered support medical staff record to produce the destination record. The destination record is stored in the destination database 28. A nonparticipation flag or decline flag "0" is displayed as the default in the participation column of each destination record. The nonparticipation flag "0" represents that the registered support medical staff does not participate in the medical support consultation.

Out of the command data, the registration ID, the support request comment, and the image IDs in the examination image list 54 are sent to the distribution server 27. The first information generator 34 of the distribution server 27 retrieves the content, of the electronic chart corresponding to the registration ID, from the electronic chart server 18 and retrieves an examination image corresponding to the image ID from the examination image server 19. Thereafter, the initial data is produced from the retrieved content of the electronic chart, the retrieved examination image, the patient ID, the registration time, the registration ID, the support request comment, and the name of the primary care physician.

The time limit setter 35 sets the time limit on the initial data. The time limit is 10 minutes from the distribution or transmission of the initial data or the support request. The distributor 37 transmits the support request including the initial data through the firewall 21 and the network 16. Under the control of the distribution controller 38, the distributor 37 transmits the support request to the address indicated by each destination record stored in the destination database 28. Namely, the support request is transmitted to each of the registered support medical staff's support terminal TS corresponding to the destination selected or specified through the destination selection section 56 on the in-hospital terminal 13. The content of the support request including the initial data is stored as the delivered data record in the delivered information database 27a.

A part of every support terminal TS is kept turned on to receive the support request. The support terminal TS corresponding to the destination receives the support request. The support terminal TS which received the support request displays the support request receiving screen 60.

The registered support medical staff, using the support terminal TS on which the support request receiving screen 60 is displayed, refers to the support request comment 61 and the chart content 62 displayed on the support request receiving screen 60. When necessary, the registered support medical staff touches or clicks on the image view button 64 of the examination image list 63 to open the examination image display screen and refer to the examination image.

The registered support medical staff decides whether to participate in the medical support consultation according to his/her schedule, experience, or the like. The participation button 65 or the decline button 66 is touched according to the decision.

For example, when the registered support medical staff using the tablet terminal TS1 as the support terminal TS has decided not to participate, he/she touches the decline button 66. In response to this, the decline notification is transmitted to the tablet terminal TS1 to the medical support apparatus 12. A change is not made to the participation column of the destination record when the medical support apparatus 12 receives the decline notification. The nonparticipation flag "0" is maintained. Subsequent remark and the like pertaining to the medical support consultation are not transmitted to the tablet terminal TS1 from which the decline notification is transmitted. After the decline notification is transmitted, the support request receiving screen 60 is closed.

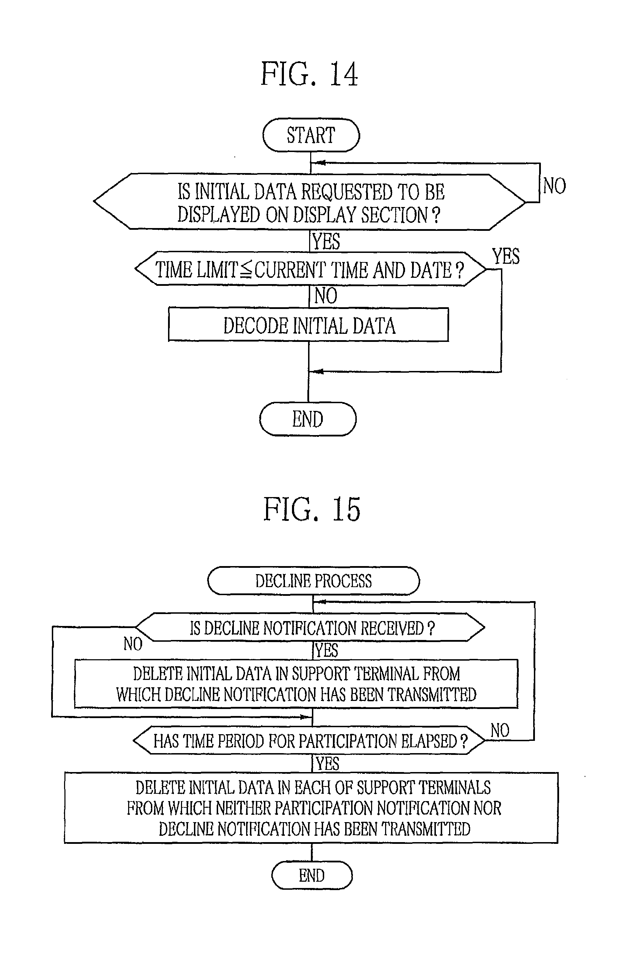

The time limit for the initial data, stored in the tablet terminal TS1 of the registered support medical staff not participating the medical support consultation, is not extended. As shown in Fig. 14, for example, if the registered support medical staff tries to refer to the initial data when 10 minutes has passed from the distribution or transmission of the support request, the time limit controller 43 compares the current time and date with the time limit for the initial data. The current time and date is the same as or exceeds the time limit, so that the initial data is not decoded. The initial data is prevented from being displayed on the display TS1a. Even if the tablet terminal TS1 is out of range of the wireless LAN for connecting to the network 16, the initial data is not displayed when the time limit is reached.

Even if a registered support medical staff not participating the medical support consultation or a third party obtains the tablet terminal TS1 illegally, the access or reference to the initial data is disabled when the time limit is reached. Thereby, leakage of medical information of the patient is prevented. The primary care physician can specify or select the destination through the support request screen 50 and transmit the support request efficiently without checking whether the registered support medical staff, being the destination, is available.

When the registered support medical staff has decided to participate, he/she touches the participation button 65. In response to this, the display TS1a displays a screen (not shown) to which the authentication ID and the password are inputted. The registered support medical staff uses the soft keyboard to input the predetermined authentication ID and password. Then, the submit button is touched.

When the submit button is touched, the tablet terminal TS1 transmits the inputted authentication ID and the password, and the participation notification to the medical support apparatus 12 through the network 16 and the firewall 21.

The management server 25 receives the authentication ID and the password from the support terminal TS (in this example, the tablet terminal TS1). The authentication section 32 of the management server 25 identifies the support terminal TS, which sent the authentication ID and the password, from the data obtained together with the authentication ID and the password. Then, the destination record having the terminal data which matches the terminal data of the identified support terminal TS is retrieved from the destination database 28. The authentication section 32 determines whether the authentication ID and the password stored in the destination record match the authentication ID and the password sent from the support terminal TS.

For example, when the authentication ID or the password does not match, a message is sent to the tablet terminal TS1 through the distribution server 27. The message notifies the user that the authentication ID or the password is incorrect and prompts the user to input the correct authentication ID or the password. Note that, when the destination record (registered support medical staff record) having the terminal data which matches the terminal data of the identified support terminal TS is not found, the support terminal TS displays that the support terminal TS in use is not registered, for example.

When the authentication IDs and the passwords match, the authentication section 32 judges that the user of the support terminal TS is a valid registered support medical staff. The registered support medical staff is approved as the participant of the medical support consultation. The participant flag "1" is written into the participation column of the destination record of the participant. The above-described process is performed every time the authentication ID, the password, and the participation notification are received from the support terminal TS.

The distribution server 27 sends notification of normal completion of authentication to the participant' s tablet terminal TS1 when the participation flag "1" is written into the participation column of the destination record corresponding to the participant's tablet terminal TS1. As shown in Fig. 8, in response to this, the display TS1a of the tablet terminal TS1 displays the remark bulletin board 68. When the participant has not made a remark, the remark bulletin board 68 displays the support request comment 71 and the thumbnail image 73 of the examination image list 54, based on the initial data.

The time limit extender 40 of the terminal control server 26 detects the change (from "0" to "1") of the participation flag when the registered support medical staff is authenticated as the participant to participate in the medical support consultation. The time limit extender 40 accesses the delivered information memory 42 of the tablet terminal TS1 whose participation flag has changed to "1" and extends the time limit for the initial data to 3 hours and 10 minutes, for example.

To make a remark, the participant touches the remark button 77 located below the remark bulletin board 68. In response to this, the remark input screen 81 is displayed. After inputting the advice and/or findings into the remark box 82, the participant touches the submit button 83. Thereby, the remark inputted is transmitted to the medical support apparatus 12.

Note that, to view the examination image after the remark bulletin board 68 is displayed, the thumbnail image 73 displayed on the remark bulletin board 68 is touched to open the examination image display screen 86. To view the content of the electronic chart, the chart view button 78 is touched to display it.

The remark from the tablet terminal TS1 is transmitted from the second information generator 36 of the distribution server 27 to the distribution controller 38. Under the control of the distribution controller 38, the remark is distributed to the respective addresses in the address data of the destination records, in the destination database 28, in each of which the participation flag in the participation column is "1", and the primary care physician's in-hospital terminal 13. The remark distributed is stored as the delivered data record in the delivered information database 27a.

Note that when the support terminal TS is the personal computer TS2, the participation/nonparticipation to the medical support consultation is sent or the examination image is viewed with a click of the mouse instead of touching. The remark is inputted by using the keyboard TS2b.

Upon receiving the remark from the distribution server 27, the display of the remark bulletin board 68 of each of the support terminal TS and the primary care physician's in-hospital terminal 13 is updated to display the received remark 72. A similar process is performed every time a remark is transmitted from the support terminal TS, to display the new remark 72. A similar process is performed every time the primary care physician operates the remark button 92 to transmit his/her remark from the primary care physician's in-hospital terminal 13.

When an examination image of the patient, being the subject of the medical support consultation, is taken using the modality 23, and the examination image server 19 detects that the patient is the subject of the medical support consultation from the patient ID, the taken examination image is sent to the distribution server 27. The examination image is transmitted from the second information generator 36 of the distribution server 27 to the distributor 37. The distributor 37 transmits the examination image to the address in the address data of each destination record with the participation flag "1" and the primary care physician's in-hospital terminal 13.

The thumbnail image 73 of the examination image is displayed on the remark bulletin board 68 of each of the support terminal TS and the in-hospital terminal 13. A click or a touch on the thumbnail image 73 opens the examination image display screen 86 that displays the examination image. Every time an examination image of the patient, being the subject of the medical support consultation, is taken using the modality 23, the examination image is transmitted and the thumbnail image 73 corresponding to the transmitted examination image is displayed on the remark bulletin board 68, in a manner similar to the above.

When the primary care physician operates the in-hospital terminal 13 to input content to the electronic chart, the electronic chart server 18 adds the inputted content to the treatment data record in the electronic chart database 18a. When it is detected that the treatment data record to which the content is added pertains to the case, based on the registration ID, the added content of the electronic chart is sent to the distribution server 27. The distribution server 27 distributes or transmits the added content to each of the participants' support terminals TS, in a manner similar to the remarks and examination images. After this, the updated contents of the electronic chart including the added content are viewed using the chart view button 78.

As the medical support consultation progresses, the primary care physician and the participants exchange remarks and opinions and the participants advise the primary care physician while referring to the remarks displayed on the remark bulletin board 68 and the examination image displayed on the examination image display screen 86. Owing to this, the primary care physician provides appropriate diagnosis and treatment.

As shown in Fig. 14, for example, when the support medical staff operates the support terminal TS to refer to the examination image and the content of the electronic chart out of the initial data during the medical support consultation, the time limit controller 43 compares the current time and date with the time limit for the initial data. When the current time and date is within the time limit, the encoded initial data is decoded and transmitted to the display section 44. Thereby, the examination image and the content of the electronic chart included in the initial data are displayed on the display TS1a or the monitor TS2a. When the current date and time is the same as the time limit or exceeds the time limit, the initial data is not decoded. The initial data is not displayed on the display TS1a and the monitor TS2a.

The time limit for the initial data stored in the support terminal TS of the participant of the medical support consultation is extended to 3 hours and 10 minutes. Within the time limit, the participant is permitted to refer to the examination image and the content of the electronic chart included in the initial data.

When the participant needs to withdraw from the medical support consultation underway, the participant touches the end button 79. Thereby, the withdrawal notification is transmitted from the participant's support terminal TS to the medical support apparatus 12. The management server 25 changes from the participation flag to the nonparticipation flag in the participation column of the destination record corresponding to the support terminal TS. Thereby, the distribution of the remarks and the like to the support terminal TS which transmitted to the withdrawal notification is stopped. The transmitted data, pertaining to the case, stored in the support terminal TS which transmitted the withdrawal notification is deleted under control of the terminal control server 26 through the network 16.

For example, the primary care physician touches the end button 93 on the in-hospital terminal 13 to end the medical support consultation when the treatment is completed with the sufficient medical support. In response to this, a notification (end notification) of the end of the medical support is sent to the medical support apparatus 12. The distribution server 27 sends the end notification to each of the participants' support terminals TS. The transmitted data, pertaining to the case, stored in each support terminal TS is deleted under control of the terminal control server 26 through the network 16.

In Fig. 15, the initial data transmitted to the support terminal TS of the registered support medical staff not participating in the medical support consultation is deleted by way of example. For example, the terminal control server 26 accesses the support terminal TS which transmitted the decline notification and deletes the initial data when the medical support apparatus 12 receives the decline notification. The application for the participation is permitted only for the predetermined time period after the transmission of the support request. The terminal control server 26 also accesses the support terminal TS which transmitted neither the participation notification nor the decline notification and deletes the initial data. In case the terminal control server 26 fails to access the support terminal TS, the time limit is set on the initial data regardless of whether the initial data is deleted after the time limit.

Various changes and modifications are possible in the present invention and may be understood to be within the present invention.

## Claims

1. An apparatus (12) for providing medical support comprising:
a first information generator (34) for producing first medical information to be transmitted at the beginning of medical support consultation for the medical support;
a time limit setter (35) for setting a time limit on the first medical information, access to the first medical information on a terminal (TS1, TS2) to which the first medical information is transmitted being disabled when the time limit is reached;
a second information generator (36) for producing second medical information after the first medical information;
a distributor (27, 37) for transmitting the first medical information on which the time limit has been set and the second medical information; and
a distribution controller (38) for controlling the distributor to transmit the first medical information to first terminals (TS1, TS2) and transmit the second medical information to at least one second terminal (TS1, TS2) out of the first terminals, the at least one second terminal transmitting notification of participation in the medical support consultation after the transmission of the first medical information.

2. The apparatus of claim 1, wherein the first terminals are used by registered support medical staffs.

3. The apparatus of claim 2, wherein the first terminals are used by the registered support medical staffs satisfying a requirement specified by a primary care physician, out of the registered support medical staffs, and the primary care physician requests the medical support.

4. The apparatus of claim 2 or 3, further comprising an authentication section (32) for judging whether a user of the second terminal is the registered support medical staff based on authentication data transmitted from the second terminal;
the distribution controller transmitting the second medical information to the second terminal whose user has been judged as the registered support medical staff by the authentication section.

5. The apparatus of one of claims claim 1 to 4, further comprising a time limit extender (40) for extending the time limit set on the first medical information in the second terminal.

6. The apparatus of one of claims 1 to 5, further comprising an information deletion section (26) for deleting the first medical information in the first terminal not transmitting the notification of participation within a predetermined time period.

7. The apparatus of one of claims 1 to 6, wherein the first medical information is transmitted together with a support request for requesting the participation in the medical support consultation.

8. A method for providing medical support comprising the steps of:
(a) producing first medical information to be transmitted at the beginning of medical support consultation for the medical support;
(b) setting a time limit on the first medical information, access to the first medical information on a terminal to which the first medical information is transmitted is disabled when the time limit is reached;
(c) transmitting the first medical information, on which the time limit has been set, to first terminals, the first thermals being used by registered support medical staffs;
(d) producing second medical information after the first medical information;
(e) transmitting the second medical information to at least one second terminal out of the first terminals, the at least one second terminal transmitting notification of participation in the medical support consultation after transmission of the first medical information.

9. The method of claim 8, wherein in the step (e), the second medical information is transmitted to the second terminal, a user of the second terminal is authenticated as the registered support medical staff based on authentication data transmitted from the second terminal.

10. The method of claim 8 or 9, further comprising the step of:
(f) extending the time limit set on the first medical information in the second terminal.

11. The method of one of claims 8 to 10, further comprising the step of:
(g) deleting the first medical information in the first terminal not transmitting the notification of participation within a predetermined time period.

12. The method of one of claims 8 to 11, wherein in the step (c), the first medical information is transmitted together with a support request for requesting the participation in the medical support consultation.
